(19) **Europäisches Patentamt**
**European Patent Office**
**Office européen des brevets**

(11) **EP 4 607 518 A1**

(12) **EUROPEAN PATENT APPLICATION**

(43) Date of publication:
**27.08.2025 Bulletin 2025/35**

(21) Application number: **25157892.8**

(22) Date of filing: **14.02.2025**

(51) International Patent Classification (IPC):
**G16B 15/20** (2019.01) **G16B 40/20** (2019.01)

(52) Cooperative Patent Classification (CPC):
**G16B 15/20; G16B 40/20;** G16B 30/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.02.2024 JP 2024024940**

(71) Applicants:
• **FUJITSU LIMITED**
**Kawasaki-shi, Kanagawa 211-8588 (JP)**

• **RIKEN**
**Wako-shi, Saitama 351-0198 (JP)**

(72) Inventors:
• **TOMA, Mitsunori**
**Kawasaki-shi, Kanagawa, 211-8588 (JP)**
• **KATOH, Takashi**
**Kawasaki-shi, Kanagawa, 211-8588 (JP)**
• **TOKUHISA, Atsushi**
**Wako-shi, Saitama, 351-0198 (JP)**

(74) Representative: **Hoffmann Eitle**
**Patent- und Rechtsanwälte PartmbB**
**Arabellastraße 30**
**81925 München (DE)**

(54) **PREDICTION CONTROL PROGRAM, INFORMATION PROCESSING APPARATUS, AND PREDICTION CONTROL METHOD**

(57) A prediction control program is a prediction control program for a structure prediction model 11 that predicts a three-dimensional structure of an organic compound from sequence information on the organic compound. The prediction control program causes a computer to execute a process of changing an intermediate feature value of the structure prediction model 11 so that a difference between a three-dimensional density map m1 and a three-dimensional density map m0 different from the three-dimensional density map m1 and actually measured is lessened, the three-dimensional density map m1 corresponding to a predicted structure output as a prediction result from the structure prediction model 11.

FIG.1

EP 4 607 518 A1

**Description**

FIELD

The embodiments discussed herein are related to a prediction control program, an information processing apparatus, and a prediction control method.

BACKGROUND

Techniques for predicting atomic structures from amino acid sequences have been disclosed (see "Highly accurate protein structure prediction with AlphaFold" (nature.com/articles/s41586-021-03819-2) and "Accelerating AlphaFold2 Inference of Protein Three-Dimensional Structure on the Supercomputer Fugaku" by Yosuke Oyama, Akihiro Tabuchi, and Atsushi Tokuhisa, from FlexScience '23: Proceedings of the 13th Workshop on AI and Scientific Computing at Scale using Flexible Computing, August 11 ,2023, Pages 1-9, (doi.org/10.1145/3589013.3596674)). For example, a machine learning model, such as AlphaFold2, OpenFold, or RoseTTAFold, outputs one typical three-dimensional structure of atomic structures (all-atom structure models) of proteins for an amino acid sequence that is input to the machine learning model.

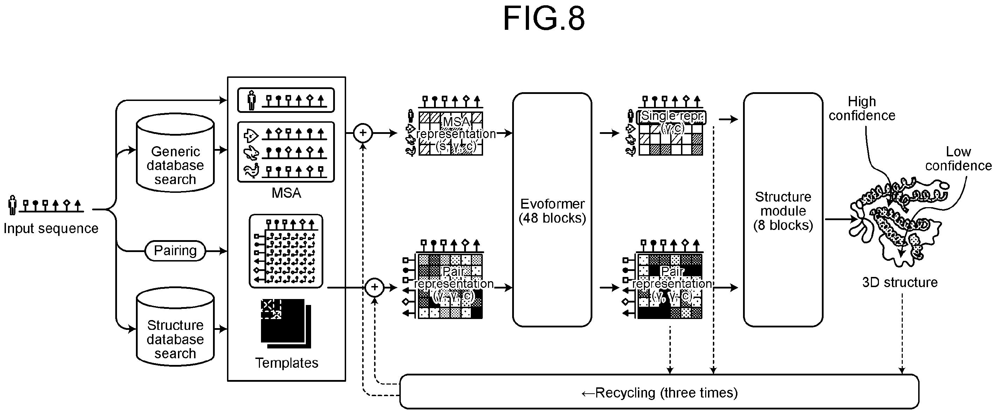

FIG. 8 is a reference diagram illustrating a machine learning model, AlphaFold2. As illustrated in FIG. 8, when an input sequence representing an amino acid sequence is input to the machine learning model, AlphaFold2, the machine learning model outputs one 3D structure that is a typical structure of a protein.

Predicting atomic structures of proteins is an important underlying technology for development of new drugs. There is a demand for prediction of diverse three-dimensional structures other than typical three-dimensional structures for application to development of new drugs.

Patent Literature 1: Japanese Laid-open Patent Publication No. 2000-229994

SUMMARY

According to an aspect of an embodiment, a prediction control program is a prediction control program of a structure prediction model that predicts a three-dimensional structure of an organic compound from sequence information on the organic compound. The prediction control program causes a computer to execute a process comprising changing an intermediate feature value of the structure prediction model so that a difference between first limiting information and second limiting information different from the first limiting information is lessened, the first limiting information corresponding to a predicted structure output as a prediction result from the structure prediction model.

BRIEF DESCRIPTION OF DRAWINGS

FIG. 1 is a diagram illustrating an image of prediction control according to a first embodiment;
FIG. 2 is a diagram illustrating an example of a functional configuration of an information processing apparatus according to the first embodiment;
FIG. 3 is a diagram illustrating an example of an extent or probabilities of existence, of an atom;
FIG. 4 is a diagram illustrating an example of display of an all-atom structure model that is output;
FIG. 5 is a diagram illustrating an example of display of coordinates of the all-atom structure model that is output;
FIG. 6 is diagram illustrating an example of a flowchart for a prediction control process according to the first embodiment;
FIG. 7 is a diagram illustrating an example of a hardware configuration; and
FIG. 8 is a reference diagram illustrating a machine learning model, AlphaFold2.

DESCRIPTION OF EMBODIMENTS

However, machine learning models according to conventional techniques only output one typical atomic structure and thus do not enable prediction of diverse three-dimensional structures.

Accordingly, it is an object in one aspect of an embodiment of the present invention to provide a computer-readable recording medium, an information processing apparatus, and a prediction control method that enable prediction of diverse three-dimensional structures.

Preferred embodiments of the present invention will be explained with reference to accompanying drawings. Each embodiment just illustrates one example or aspect, and ranges and usage scenes of numerical values and functions, for example, are not to be limited by such illustration. The embodiments may be combined, as appropriate, so long as no contradictions in processing arise from the combination.

(a) First Embodiment

Image of Prediction Control

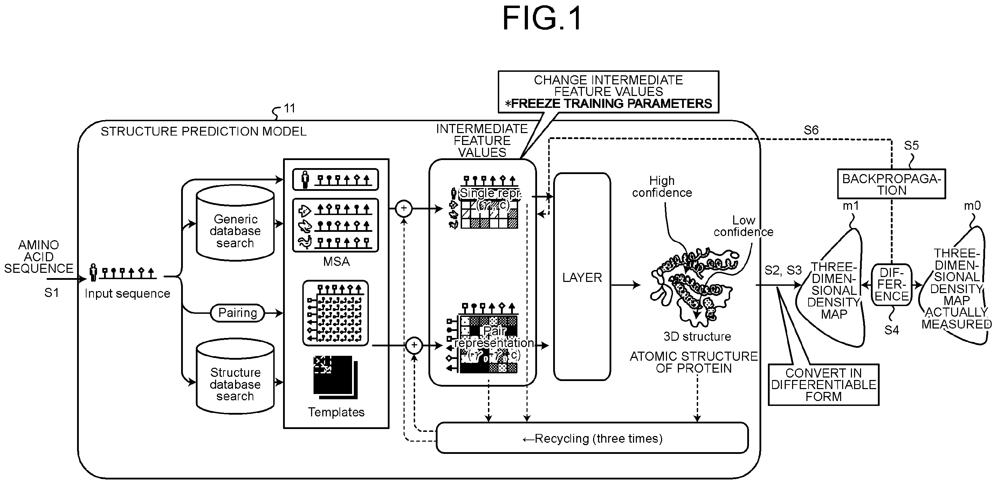

An image of prediction control according to a first embodiment will be described first by reference to FIG. 1. FIG. 1 is a diagram illustrating the image of the prediction control according to the first embodiment. A prediction control process illustrated in FIG. 1 is for predicting an atomic structure by changing intermediate feature values of a structure prediction model so as to adapt the intermediate feature values to information limiting a target atomic structure. An atomic structure to be predicted in the first embodiment may be referred to as an all-atom structure model. Information limiting an all-atom structure model may hereinafter be referred to as limiting information.

A protein will hereinafter be mentioned as an example of a target, but the target may be not a protein. For example, the target may be an organic compound other than a protein, for example, a high-polymer material. Furthermore, a three-dimensional density map limiting an all-atom structure model of a protein will be described as an example of information (limiting information) limiting a target all-atom structure model, but the information may be not a three-dimensional density map.

As illustrated in FIG. 1, the prediction control process involves a structure prediction model 11. The structure prediction model 11 referred to herein may be, for example, a machine learning model, such as AlphaFold2, OpenFold, or RoseTTAFold. In the first embodiment, AlphaFold2 is used as the structure prediction model 11, but the structure prediction model 11 to be used in the first embodiment may be not AlphaFold2.

In the prediction control process, sequence information on an organic compound is input to the structure prediction model 11. For example, in the prediction control process, amino acid sequence information is input to the structure prediction model 11, the amino acid sequence information being an example of the sequence information on the organic compound (S1). The structure prediction model 11 then extracts intermediate feature values from the amino acid sequence information, inputs the intermediate feature values extracted, to a layer, and outputs a prediction result that is an all-atom structure model (a three-dimensional structure) of a protein (S2). For convenience of description, the structure prediction model 11 has a single layer but the structure prediction model 11 may have more than one layer.

In the prediction control process, the all-atom structure model output as the prediction result is converted to a three-dimensional density map that limits the all-atom structure model, in a differentiable form (S3).

In the prediction control process, a difference between the three-dimensional density map corresponding to the all-atom structure model that is the prediction result and a three-dimensional density map that has been actually measured is calculated (S4). The three-dimensional density map that has been actually measured is, for example, a three-dimensional density map reconstructed from an electron microscope (EM) image captured by an electron microscope, such as a cryo-electron microscope, the three-dimensional density map limiting an all-atom structure model of the protein, but without being limited to this example, the three-dimensional density map that has been actually measured may be any three-dimensional density map having a three-dimensional voxel data format.

In the prediction control process, backpropagation of the difference is then performed, and intermediate feature values that would minimize the difference are thereby calculated (S5). In the prediction control process, the intermediate feature values of the structure prediction model 11 are then updated with the intermediate feature values calculated (S6). That is, in the prediction control process, the intermediate feature values of the structure prediction model 11 are changed so that the difference between the three-dimensional density map corresponding to the all-atom structure model that is the prediction result and the three-dimensional density map that has been actually measured is lessened. In other words, in the prediction control process, the intermediate feature values of the structure prediction model 11 are changed according to constraints from the three-dimensional density map that has been actually measured.

Thereafter, by using the intermediate feature values updated, the structure prediction model 11 predicts an all-atom structure model of the protein. Repeating S2 to S6 in the prediction control process enables prediction of diverse three-dimensional structures.

In a case where the structure prediction model 11 has plural layers, intermediate feature values to be input to any one of the plural layers may be updated in the prediction control process. In the prediction control process, the intermediate feature values of the structure prediction model 11 are updated but training parameters of the structure prediction model 11 are not updated. This is because not destroying the existing training parameters in retraining of the structure prediction model 11 prevents the structure prediction model 11 from forgetting knowledge that the structure prediction model 11 has had.

Functional Configuration of Information Processing Apparatus

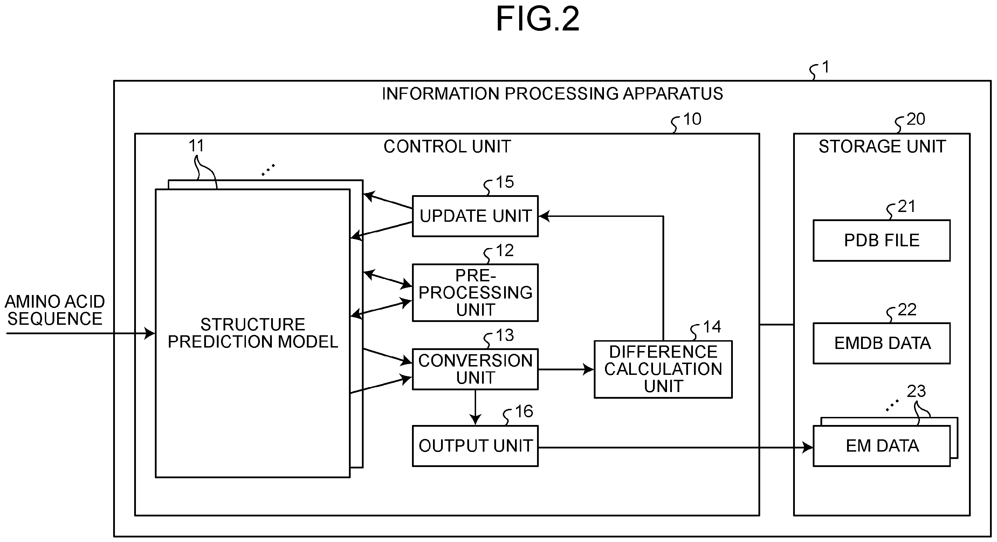

FIG. 2 is a diagram illustrating an example of a functional configuration of an information processing apparatus according to the first embodiment. An information processing apparatus 1 illustrated in FIG. 2 is an example of a computer that executes the prediction control process. As illustrated in FIG. 2, the information processing apparatus 1 has a control unit 10 and a storage unit 20.

The storage unit 20 has a protein data bank (PDB) file 21, an electron microscope data bank (EMDB) data 22, and EM data 23.

The PDB file 21 is a file having information accumulated therein, the information being on three-dimensional structures of proteins. The PDB file 21 includes, for example, information on all-atom structure models of three monomers composing each protein, and also includes information on which chain each atom belongs to. The PDB file 21 may be obtained from a PDB on the Web.

The EMDB data 22 are a file storing, as voxel data, a three-dimensional density map of each protein reconstructed from a two-dimensional EM image captured by an electron microscope, such as a cryo-electron microscope. The EMDB data 22 may be obtained from, for example, an EMDB on the Web.

The EM data 23 are a three-dimensional density map calculated from an all-atom structure model predicted by the prediction control process. The EM data 23 are stored in the storage unit 20 by an output unit 16 described later.

The control unit 10 has a plurality of the structure prediction models 11, a preprocessing unit 12, a conversion unit 13, a difference calculation unit 14, an update unit 15, and the output unit 16.

The structure prediction models 11 predict an all-atom structure model of a protein from amino acid sequence information. Each of the structure prediction models 11 is applied to one monomer. In a case where the amino acid sequence information is on a multimer, each of the structure prediction models 11 predicts an all-atom structure model of a monomer composing the protein for each chain. A case where the target protein is a trimer will be described with respect to the first embodiment.

The preprocessing unit 12 executes preprocessing of the prediction control.

In an example of first preprocessing, the preprocessing unit 12 finds, through point set registration, three rigid body transformations respectively fitting chains of all-atom structure models of three monomers (a trimer) predicted, by using the PDB file 21. This is performed in order to find the rigid body transformations corresponding to the separate chains. The PDB file 21 includes information on all-atom structure models of three monomers composing each protein, and also includes information on which chain each atom belongs to. Therefore, by using the PDB file 21, the preprocessing unit 12 is able to perform the point set registration for each of the chains of the all-atom structure models of the trimer predicted and is thus able to find the three rigid body transformations matching the chains. The preprocessing unit 12 then applies the rigid body transformations found, to the chains of the all-atom structure models of the three monomers predicted and performs combination into one multimer.

In an example of second preprocessing, the preprocessing unit 12 determines rigid body transformations by which the combined multimer and a target three-dimensional density map in the EMDB data 22 are fitted to each other most. That is, the preprocessing unit 12 determines rigid body transformations used for registration between the all-atom structure model and the limiting three-dimensional density map. An equation for the registration is expressed by, for example, Equation (1). In Equation (1), $R_c$ and $t_c$ are rigid body transformations representing rotation and translation. In Equation (1), $x_{ca}$ and $x'_{ca}$ represent an atomic coordinate of the original atom a and an atomic coordinate of the atom a after the rigid body transformations.

$$x'_{ca} = R_c x_{ca} + t_c \qquad \cdots \text{Equation (1)}$$

For example, in the second preprocessing the preprocessing unit 12 performs a first step and a second step. At the first step, by using the limiting three-dimensional density map, the preprocessing unit 12 performs barycenter alignment (translation) and principal component alignment (rotation) for the combined multimer and roughly determines $t_c$ and $R_c$. At the second step, on the basis of Equation (1), the preprocessing unit 12 finely adjusts the registration, with $R_c$ and $t_c$ serving as variables and $x_{ca}$ serving as a constant (fixed), to determine $t_c$ and $R_c$. That is, in the second preprocessing, how a rigid body is to be translated and rotated to fit the limiting three-dimensional density map most is found, the rigid body being the one multimer that is a combination of the monomers, without use of information on which chains the atoms of the monomers originally belonged to. The preprocessing unit 12 is thereby able to perform registration between the all-atom structure model and the limiting three-dimensional density map.

After the preprocessing by the preprocessing unit 12 is ended, the control unit 10 causes forward propagation through the layer of the structure prediction model 11 for each chain. A functional unit to cause the forward propagation may be not the control unit 10 and may be the structure prediction models 11. Or the forward propagation may be performed by a user. An equation for causing forward propagation through the layer is expressed by, for example, Equation (2). In Equation (2), $r_{ci}$ represents an intermediate feature value for each chain. In Equation (2), i is an index indicating each residue. In Equation (2), $x_{ca}$ represents the atomic coordinate of the atomic structure predicted. In a case where forward propagation through the layer is performed, the intermediate feature values of the structure prediction model 11 are not changed.

$$x_{ca} = \text{StructureModule}(r_{ci}) \qquad \cdots\text{Equation (2)}$$

The second preprocessing is divided into two steps, the first step and the second step, for the determination of the rigid body transformations by which the combined multimer and the limiting three-dimensional density map are fitted to each other most. However, in the second preprocessing, without being limited to this example, the rigid body transformations by which the combined multimer and the limiting three-dimensional density map are fitted to each other most may be determined by use of a genetic algorithm.

The conversion unit 13 converts atomic structures output as prediction results from the structure prediction models 11 to a three-dimensional density map (EM) limiting the all-atom structure model, by differentiable calculation.

For example, the conversion unit 13 applies rigid body transformations found by the preprocessing unit 12 to an all-atom structure model that is a prediction result, to find an atomic structure of one multimer (a trimer). That is, by using Equation (1), the conversion unit 13 finds the all-atom structure model of the one multimer (trimer) from the all-atom structure model that is the prediction result.

By using interpolation formulae satisfying the density conservation law, the conversion unit 13 converts the prediction result that is the all-atom structure model of the trimer, to a three-dimensional density map limiting the all-atom structure model. The interpolation formulae satisfying the density conservation law are expressed by, for example, the following Equation (3), Equation (4), and Equation (5).

In Equation (3), $d_N$ represents a length of one side of a voxel. In Equation (3), $x'_{Ngk}$ and $x'_{cak}$ respectively represent a k-component of coordinates of a vertex of the voxel and a k-component of atomic coordinates. In Equation (3), $u_{Ngcak}$ represents a distance between an atom a and a vertex g of the voxel, the distance having been normalized with $d_N$.

$$u_{Ngcak} = \frac{\left| x'_{cak} - x'_{Ngk} \right|}{d_N} \qquad \cdots\text{Equation (3)}$$

In Equation (4), $u_{Ngcak}$ represents the distance between the atom a and the vertex g of the voxel, the distance having been normalized with $d_N$, the distance being a result of calculation using Equation (3). In Equation (4), $p_{Ngcak}$ represents a probability of existence of the atom a at the vertex g of the voxel.

$$p_{Ngcak} = \begin{cases} \frac{1}{8} u_{Ngcak}^3 - \frac{3}{8} u_{Ngcak}^2 + \frac{1}{2} & \text{if } u_{Ngcak} < 2 \\ 0 & \text{otherwise} \end{cases} \qquad \cdots\text{Equation (4)}$$

In Equation (5), $n_a$ represents the atomic number of the atom a. In Equation (5), $p_{Ngcak}$ represents the probability of existence of the atom a at the vertex g of the voxel, the probability being a result of calculation using Equation (4). In Equation (5), $\rho_{Ng}^{pred}$ represents a three-dimensional density map calculated.

$$\rho_{Ng}^{\text{pred}} = \sum_c \sum_a n_a \prod_k p_{Ngcak} \qquad \cdots\text{Equation (5)}$$

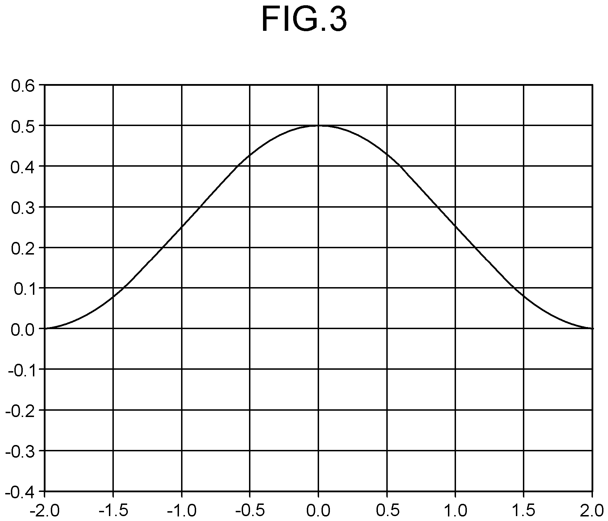

FIG. 3 illustrates a relation between the distance $u_{Ngcak}$ between the atom a and the vertex g of the voxel, expressed by Equation (3), and the probability of existence $P_{Ngcak}$ of the atom a at the vertex g of the voxel, expressed by Equation (4). FIG. 3 is a diagram illustrating an extent or probabilities of existence, of an atom. The x-axis of the graph illustrated in FIG. 3 represents a value group obtained from an equation resulting from removal of the absolute value symbol of the numerator expressed on the right hand side of Equation (3). That is, the x-axis represents a value group normalized to indicate how close the atom is to the vertex of the voxel. The y-axis represents the probability of existence $p_{Ngcak}$ of the atom a at the vertex g of the voxel, expressed by Equation (4). This graph illustrates that the closer the atom is to the vertex of the voxel (the closer the value on the x-axis is to 0), the higher the probability of existence of the atom, and the farther the atom is to the vertex of the voxel (the farther the value on the x-axis is from 0), the lower the probability of existence of the atom. If the value on the x-axis is larger than "2" or smaller than "-2", the probability of existence of the atom on the y-axis is "0". Therefore, by using the probability of existence $p_{Ngcak}$ of the atom a at the vertex g of the voxel found by Equation (3) and Equation (4), the conversion unit 13 enables a conversion to a smooth image (a three-dimensional density map) where the atoms look hazy (a concept of a filter). That is, the conversion unit 13 implements a conversion process having an extent of an atom and a concept of a filter integrated into one.

The conversion unit 13 thus converts an atomic structure of a trimer that is a prediction result into a three-dimensional density map $\rho_{Ng}^{pred}$ limiting the atomic structure by using Equation (3), Equation (4), and Equation (5). These Equation (3), Equation (4), and Equation (5) are functions for a conversion from an all-atom structure model to a three-dimensional density map limiting the all-atom structure model and are differentiable conversion functions.

The difference calculation unit 14 calculates a difference between: a three-dimensional density map corresponding to an all-atom structure model of a trimer from a prediction result; and a target three-dimensional density map in the EMDB data 22. For example, by finding a cross-correlation, the difference calculation unit 14 calculates a difference between: a three-dimensional density map limiting an all-atom structure model from a prediction result; and a target three-dimensional density map. For example, the following Equation (6), for example, is an equation for finding the cross-correlation.

In Equation (6), $\rho_{Ng}^{pred}$ represents a three-dimensional density map calculated by Equation (3), Equation (4), and Equation (5). In Equation (6), $\rho_{Ng}^{targ}$ represents a target three-dimensional density map. In Equation (6), $L_N$ represents a cross-correlation value for the three-dimensional density maps. In Equation (6), g and N respectively represent a vertex of a voxel and the number of divisions for the voxel.

$$L_N = 1 - \frac{\sum_g^{N^3} \rho_{Ng}^{pred} \rho_{Ng}^{targ}}{\sqrt{\sum_g^{N^3} \left(\rho_{Ng}^{pred}\right)^2} \sqrt{\sum_g^{N^3} \left(\rho_{Ng}^{targ}\right)^2}} \qquad \cdots \text{Equation (6)}$$

The difference calculation unit 14 thus calculates a difference between: a three-dimensional density map of the atoms a of a trimer from a prediction result and a target three-dimensional density map, by using Equation (6). This Equation (6) is a differentiable equation. The equation for calculating the difference has been described as the equation for finding the cross-correlation but the equation for calculating the difference is not limited to this example. An L2 norm or an L1 norm may be used as the equation for calculating the difference.

Furthermore, by using the difference calculated, the difference calculation unit 14 calculates an objective function indicating a constraint on the all-atom structure model of the trimer from the prediction result. In addition, the difference calculation unit 14 adds a constraint that the protein is supposed to have to the objective function calculated and calculates a final objective function. For example, the difference calculation unit 14 calculates the final objective function by using the following Equation (7).

In Equation (7), $L_N$ is the value calculated by Equation (6). In Equation (7), $L_{bondlength}$ and $L_{bondangle}$ are respectively examples of objective functions for maintaining a distance and an angle of a peptide bond. In Equation (7), N represents the number of divisions for the voxel. In Equation (7), $L_{total}$ is the final objective function.

$$L_{total} = \frac{\sum_{N=8}^{128} N^{-1} L_N}{\sum_{N=8}^{128} N^{-1}} + \beta_1 L_{bondlength} + \beta_2 L_{bondangle} \qquad \cdots \text{Equation (7)}$$

The first term on the right hand side of Equation (7) is the main objective function. This first term reduces the difference smoothly by using a coarse and dense mixing technique for area segmentation.

By using Equation (7), the difference calculation unit 14 calculates an objective function indicating constraints on the atomic structure of the trimer from the prediction result. This Equation (7) is a differentiable equation. The constraints to be added have been described as the distance and angle of the peptide bond, for example, but without being limited to this example, the constraints to be added may be any of the excluded volume effect, the disulfide bond distance, and the hydrogen bond energy. In effect, the difference calculation unit 14 may add, as options, constraints that the protein is supposed to have, to the main objective function.

The control unit 10 then performs calculation for backpropagation of the difference. The calculation for the backpropagation of the difference may be not performed by the control unit 10 and may be performed by, for example, the difference calculation unit 14. For example, the control unit 10 performs the calculation for the backpropagation of the difference by using the following Equation (8) to Equation (13). Equation (8) differentiates $L_{total}$ expressed by Equation (7) with respect to $L_N$. Equation (9) differentiates $L_N$ expressed by Equation (6) with respect to $\rho_{Ng}^{pred}$. Equation (10) differentiates $\rho_{Ng}^{pred}$ expressed by Equation (5) with respect to $\rho_{Ngcak}$. Equation (11) differentiates $\rho_{Ngcak}$ expressed by Equation (4) with respect to $u_{Ngcak}$. Equation (12) differentiates $u_{Ngcak}$ expressed by Equation (3) with respect to $x'_{cak}$.

Equation (13) differentiates $L_N$ expressed by Equation (6) with respect to $x'_{cak}$.

$$\frac{\partial L_{\text{total}}}{\partial L_N} = \frac{N^{-1}}{\sum_{N=8}^{128} N^{-1}} \quad \cdots \text{Equation (8)}$$

$$\frac{\partial L_N}{\partial \rho_{Ng}^{\text{pred}}} = \frac{\left(\rho_{Ng}^{\text{pred}} / \sum_g^{N^3} \left(\rho_{Ng}^{\text{pred}}\right)^2\right)\sum_g^{N^3} \rho_{Ng}^{\text{pred}}\rho_{Ng}^{\text{targ}} - \rho_{Ng}^{\text{targ}}}{\sqrt{\sum_g^{N^3}\left(\rho_{Ng}^{\text{pred}}\right)^2}\sqrt{\sum_g^{N^3}\left(\rho_{Ng}^{\text{targ}}\right)^2}} \quad \cdots \text{Equation (9)}$$

$$\frac{\partial \rho_{Ng}^{\text{pred}}}{\partial p_{Ngcak}} = n_a \prod_{k' \neq k} p_{Ngcak'} \quad \cdots \text{Equation (10)}$$

$$\frac{\partial p_{Ngcak}}{\partial u_{Ngcak}} = \begin{cases} \frac{3}{8}u_{Ngcak}^2 - \frac{3}{4}u_{Ngcak} & \text{if } u_{Ngcak} < 2 \\ 0 & \text{otherwise} \end{cases} \quad \cdots \text{Equation (11)}$$

$$\frac{\partial u_{Ngcak}}{\partial x'_{cak}} = \frac{\text{sgn}\left(x'_{cak} - x'_{Ngk}\right)}{d_N} \quad \cdots \text{Equation (12)}$$

$$\frac{\partial L_N}{\partial x'_{cak}} = \sum_g \frac{\partial L_N}{\partial \rho_{Ng}^{\text{pred}}}\frac{\partial \rho_{Ng}^{\text{pred}}}{\partial p_{Ngcak}}\frac{\partial p_{Ngcak}}{\partial u_{Ngcak}}\frac{\partial u_{Ngcak}}{\partial x'_{cak}} \quad \cdots \text{Equation (13)}$$

Furthermore, the control unit 10 calculates a gradient according to an intermediate feature value of $L_{\text{total}}$ for each chain by backpropagation of the difference. This calculation of the gradient may be not performed by the control unit 10 and may be performed by the difference calculation unit 14 in a case where the backpropagation of the difference is performed by the difference calculation unit 14. Or the calculation of the gradient may be performed by means of the structure prediction models 11 or the conversion unit 13.

The update unit 15 calculates an intermediate feature value for each chain using the following Equation (14), by using the gradient calculated by the backpropagation of the difference. In Equation (14), $r_{ci}$ represents the intermediate feature value for each chain. In Equation (14), i is an index indicating each residue. In Equation (14), $\delta x_{ca}/\delta r_{ci}$ is a differential of the atomic coordinate $x_{ca}$ expressed by Equation (2) with respect to the intermediate feature value $r_{ci}$ of each chain.

$$r_{ci} \leftarrow r_{ci} - \eta \sum_a \frac{\partial L}{\partial x_{ca}}\frac{\partial x_{ca}}{\partial r_{ci}} \quad \cdots \text{Equation (14)}$$

The update unit 15 updates the structure prediction model 11 corresponding to each chain, with the intermediate feature value for that chain. That is, the update unit 15 changes the intermediate feature values of the structure prediction models 11 so that the difference between the all-atom structure model from the prediction result and the target all-atom structure model is lessened. In other words, the update unit 15 changes the intermediate feature values of the structure prediction models 11 according to the constraints from the limiting three-dimensional density map.

The output unit 16 stores, as the EM data 23, the three-dimensional density map (EM) converted by the conversion unit 13, into the storage unit 20.

Example of Display of Atomic Structure Output

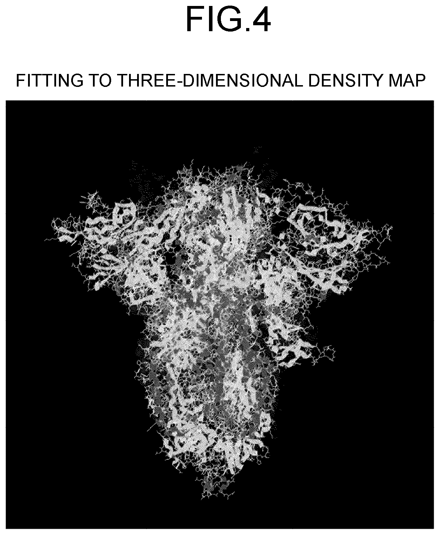

FIG. 4 is a diagram illustrating an example of display of an all-atom structure model output. FIG. 4 illustrates an image displayed on a screen, the image being an image of the atomic coordinates $x'_{ca}$ (see Equation (1)) of the all-atom structure model output from the structure prediction models 11. This atomic coordinates $x'_{ca}$ of the all-atom structure model are atomic coordinates after a rigid body transformation to each chain has been performed. By operating the atomic coordinates of the all-atom structure model displayed on the screen, a user is able perform fitting (adjustment) to a three-dimensional density map.

With respect to the first embodiment, the limiting information limiting the all-atom structure model has been described as a three-dimensional density map (EM). However, the limiting information limiting the all-atom structure model may be not a three-dimensional density map (EM) and may be a three-dimensional density map obtained by X-ray structure analysis. Or the limiting information limiting the all-atom structure model may be coordinates of the all-atom structure model.

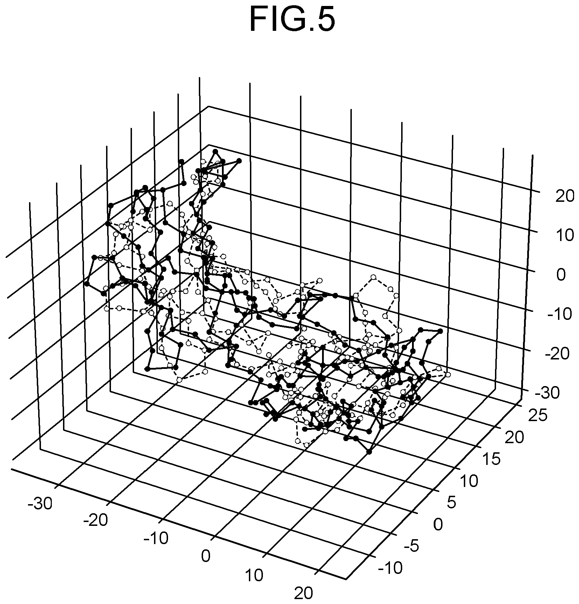

An example of display of an all-atom structure model output in a case where limiting information limiting the all-atom structure model is coordinates of the all-atom structure model will be described by reference to FIG. 5. FIG. 5 is a diagram illustrating an example of display of coordinates of the all-atom structure model output. FIG. 5 illustrates an image (solid lines) displayed on a screen, the image being an image of coordinates of an all-atom structure model output from the structure prediction models 11. These atomic coordinates of the all-atom structure model are coordinates after a rigid body transformation to each chain has been performed. In addition, FIG. 5 illustrates an image (broken lines) displayed on the screen, the image being an image of coordinates of limiting information. In a case where the limiting information limiting the all-atom structure model is coordinates of an atomic structure, target limiting information may be obtained from, for example, the PDB file 21. A user is able to use the coordinates (solid lines) of the all-atom structure model output from the structure prediction models 11 in fitting to the coordinates (broken lines) of the limiting information.

In FIG. 5, only carbon atoms ($C\alpha$) present in the principal chain of each amino acid are displayed. The number of the carbon atoms ($C\alpha$) is about 1/10 of the total number of atoms. The structure of the atomic coordinates and lines in the all-atom structure model output from the structure prediction models 11 has, not only the carbon atoms ($C\alpha$) being displayed, but also positional information on all of the atoms. However, the structure of the coordinates and lines of the target limiting information is obtained from the PDB file 21 and also includes positional information on atoms other than the carbon atoms ($C\alpha$), but only the carbon atoms ($C\alpha$) are used as the limiting information. That is, even if the atomic structure that is able to be used as the limiting information has only about 1/10 of all of the atoms, a user is able to restore positional information on all of the atoms by having, as a clue, the all-atom structure model output from the structure prediction models 11.

Flowchart of Prediction Control Process

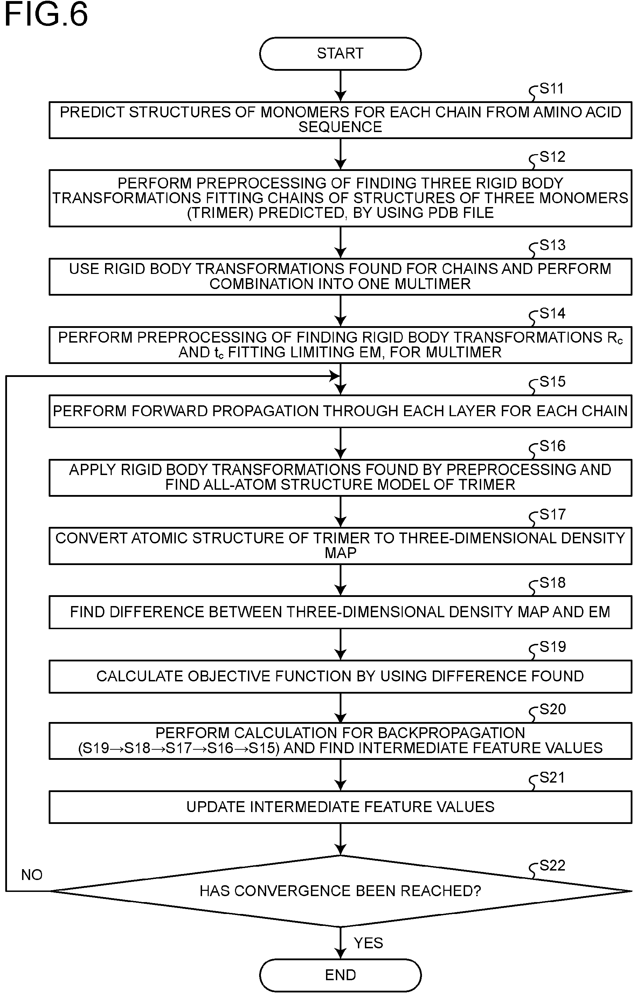

A flowchart of the prediction control process implemented by the information processing apparatus 1 will hereinafter be described by use of FIG. 6. FIG. 6 is a diagram illustrating an example of the flowchart of the prediction control process according to the first embodiment.

As illustrated in FIG. 6, the information processing apparatus 1 predicts a structure of monomers for each chain from an amino acid sequence (Step S11). For example, the structure prediction models 11 predict all-atom structure models of monomers composing a protein for respective chains from an amino acid sequence.

As preprocessing, the information processing apparatus 1 finds three rigid body transformations fitting chains of structures of three monomers (trimer) predicted, by using the PDB file 21 (Step S12). For example, the preprocessing unit 12 executes first preprocessing. The information processing apparatus 1 then uses the rigid body transformations found respectively for the chains and combines them into a single multimer (Step S13).

As the preprocessing, the information processing apparatus 1 then finds rigid body transformations $R_c$ and $t_c$ fitting the limiting three-dimensional density map (EM) for the multimer (Step S14). For example, the preprocessing unit 12 executes second preprocessing.

The information processing apparatus 1 then causes forward propagation through each layer for each chain (Step S15). For example, the information processing apparatus 1 executes Equation (2). In executing Equation (2), the information processing apparatus 1 does not let the intermediate feature values $r_{ci}$ change.

The information processing apparatus 1 then finds an all-atom structure model of the trimer by application of the rigid body transformations found through the preprocessing at Step S12 and Step S14 (Step S16). For example, the information processing apparatus 1 executes Equation (1).

The information processing apparatus 1 then converts the atomic structure of the trimer into a three-dimensional density map (Step S17). For example, the information processing apparatus 1 executes Equation (3), Equation (4), and Equation (5).

The information processing apparatus 1 then finds a difference between the converted three-dimensional density map and a target three-dimensional density map (EM) (Step S18). For example, the information processing apparatus 1 executes Equation (6).

The information processing apparatus 1 then calculates an objective function by using the difference found (Step S19). For example, the information processing apparatus 1 executes Equation (7).

The information processing apparatus 1 then performs calculation for backpropagation (S19 → S18 → S17 → S16 → S15) and thereby finds intermediate feature values (Step S20). For example, by executing Equation 8 to Equation (14), the information processing apparatus 1 performs calculation for backpropagation and thereby finds intermediate feature values. The information processing apparatus 1 then updates the structure prediction models 11 with the intermediate feature values found (Step S21).

The information processing apparatus 1 then determines whether or not structure prediction by the structure prediction models 11 has converged (Step S22). In a case where the information processing apparatus 1 determines that the structure prediction has not converged (Step S22; No), the information processing apparatus 1 proceeds to Step S15 to implement the next structure prediction.

In a case where the information processing apparatus 1 determines that the structure prediction has converged (Step S22; Yes), the information processing apparatus 1 ends the prediction control process.

As described above, the information processing apparatus 1 according to the first embodiment changes intermediate features values of the structure prediction models 11 so that a difference between a three-dimensional density map and a different three-dimensional density map different from the three-dimensional density map is lessened, the three-dimensional density map resulting from a conversion of a predicted structure by use of interpolation formulae satisfying the density conservation law, the predicted structure being output as a prediction result from the structure prediction model 11. Therefore, the information processing apparatus 1 according to the first embodiment enables prediction of diverse three-dimensional structures. By changing the intermediate feature values of the structure prediction models 11 so that the difference between the three-dimensional density map resulting from the conversion of the predicted structure and the three-dimensional density map of the correct answer is lessened, the information processing apparatus 1 enables prediction of diverse three-dimensional structures in a practical period of time.

(b) Second Embodiment

According to the above description, the conversion unit 13 in the information processing apparatus 1 according to the first embodiment converts an atomic structure output as a prediction result from the structure prediction models 11 into a three-dimensional density map (EM) limiting the atomic structure, by using interpolation formulae satisfying the density conservation law in a differentiable form. However, without being limited to the interpolation formulae satisfying the density conservation law, the conversion unit 13 may use, for example, approximate equations according to spherically symmetric Gaussian distributions to perform a conversion to a three-dimensional density map (EM) limiting the all-atom structure model.

In a case where limiting information limiting the all-atom structure model is on a shape of the molecule, a three-dimensional electron density map of the molecule may be used. With the three-dimensional electron density map of the molecule, accurate calculation rooted in physics is enabled by use of atomic positions and the atomic scattering factor that are given.

However, the three-dimensional electron density map has many undulations and search using the three-dimensional electron density map as the limiting information may reach a plateau with a local solution, for example. Approximating the functional form of the atomic scattering factor and performing smoothing of a three-dimensional electron density map converted from the approximated functional form beforehand thus enable improvement in smoothness of the search while maintaining accuracy of the molecular form.

An atomic scattering factor f(q) is represented by four Gaussian functions and a constant term as expressed by Equation (15). The atomic scattering factor f(q) is able to be approximated to be represented by four or less Gaussian functions as expressed by Equation (16). In Equation (16), $N_f$ indicates the number of Gaussian functions to be used in the approximation. In these equations, a, b, and c are fitting parameters and fitting may be performed newly.

$$f(q) = \sum_{i=1}^{4} a_i \exp\left(-b_i \left(\frac{q}{4\pi}\right)^2\right) + c \quad \cdots \text{Equation (15)}$$

$$f(q) = \sum_{i=1}^{N_f} a_i \exp\left(-b_i \left(\frac{q}{4\pi}\right)^2\right) \quad \cdots \text{Equation (16)}$$

A low-pass filter having a cutoff wave number of $f_c$ in wave vector space may be used in an example of a method of performing smoothing of the three-dimensional electron density map, that is, making the three-dimensional electron density map into a smooth image. In a case where the low-pass filter in wave vector space is used, the number N of voxels per dimension is able to be given by the following Equation (17), where R (Å) represents an objective resolving power (the extent of smoothing) and L (Å) represents a molecular size parameter.

$$N = 2L/R = Lf_c \hspace{4cm} \text{Equation (17)}$$

The molecular size parameter L is able to be estimated precisely by using a three-dimensional electron density map that has been calculated accurately and a molecular surface defined therefrom.

The conversion unit 13 thus converts an all-atom structure model of a trimer from a prediction result into a three-dimensional density map limiting the all-atom structure model, by using Equation (16) and Equation (17). This Equation (16) is a function for a conversion from the all-atom structure model to the three-dimensional density map limiting the all-atom structure model and is a differentiable conversion function.

The information processing apparatus 1 according to the second embodiment thereby changes intermediate features values of the structure prediction models 11 so that a difference between a three-dimensional density map and a different three-dimensional density map different from the three-dimensional density map is lessened, the three-dimensional density map resulting from a conversion of a predicted structure by use of approximate equations according to spherically symmetric Gaussian distributions, the predicted structure being output as a prediction result from the structure prediction model 11. Therefore, the information processing apparatus 1 according to the second embodiment enables prediction of diverse three-dimensional structures. By changing the intermediate feature values of the structure prediction models 11 so that the difference between the three-dimensional density map resulting from the conversion of the predicted structure and the three-dimensional density map of the correct answer is lessened, the information processing apparatus 1 enables prediction of diverse three-dimensional structures in a practical period of time.

(c) Third Embodiment

Although the embodiments related to the disclosed apparatus have been described above, the present invention may be implemented in various different modes, in addition to the above described embodiments. Therefore, the following description is on some other embodiments included in the present invention.

The processing sequence, control sequence, specific names, and information including various data and parameters, which are in the above description of the first and second embodiments and illustrated in the drawings, may be modified in any way unless specifically stated otherwise.

Specific modes of separation and integration of the components of each apparatus are not limited to those illustrated in the drawings. That is, all or part of these components may be functionally or physically separated or integrated in any units according to various loads and use situations. In addition, all or any part of the processing functions of each apparatus may be implemented by a CPU and a program analyzed and executed by the CPU, or may be implemented as hardware by wired logic. Instead of the CPU or in addition to the CPU, a processor, such as a graphics processing unit (GPU) or a tensor processing unit (TPU), may be used for all or any part of the processing functions of each apparatus.

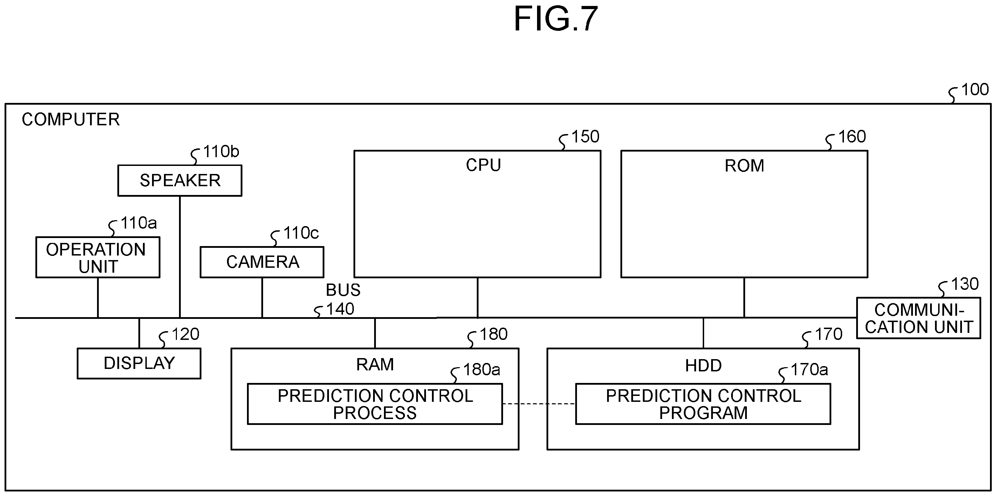

The various processes described above with respect to the first or second embodiments may be implemented by a computer, such as a personal computer or a workstation, executing a program that has been prepared beforehand. Or a so-called supercomputer or a high performance computing (HPC) machine may be used as the computer to execute the various processes described above with respect to the first or second embodiment. An example of a computer that executes a prediction control program having the same functions as the first or second embodiment will be described by use of FIG. 7.

FIG. 7 is a diagram illustrating an example of a hardware configuration. As illustrated in FIG. 7, a computer 100 has an operation unit 110a, a speaker 110b, a camera 110c, a display 120, and a communication unit 130. The computer 100 also has a CPU 150, a ROM 160, an HDD 170, and a RAM 180. These units 110 to 180 are connected to one another via a bus 140.

The HDD 170 has, stored therein, as illustrated in FIG. 7, a prediction control program 170a exhibiting the same functions as the structure prediction models 11, the preprocessing unit 12, the conversion unit 13, the difference calculation unit 14, the update unit 15, and the output unit 16 (in other words, the control unit 10), which have been described above with respect to the first embodiment. This prediction control program 170a may be integrated or separated, similarly to the structure prediction models 11, the preprocessing unit 12, the conversion unit 13, the difference calculation unit 14, the update unit 15, and the output unit 16, illustrated in FIG. 2. That is, not all of data described above with respect to the first embodiment may be stored in the HDD 170 and data used in the processing may just be stored in the HDD 170.

In such an environment, the CPU 150 reads the prediction control program 170a from the HDD 170 and loads the read prediction control program 170a into the RAM 180. As a result, the prediction control program 170a functions as a prediction control process 180a, as illustrated in FIG. 7. This prediction control process 180a loads various data read from the HDD 170 into part of a storage area that the RAM 180 has, the part being an area that has been allocated to the prediction control process 180a, and the prediction control process 180a executes various processes using the various data loaded. Examples of the processes executed by the prediction control process 180a may include the process illustrated in FIG. 6. Not all of the processing units described above with respect to the first embodiment may operate and processing units corresponding to processes to be executed may just be implemented virtually by means of the CPU 150.

The prediction control program 170a may be not stored initially in the HDD 170 or the ROM 160. For example, the prediction control program 170a is stored in a "portable physical medium", such as a flexible disk that is a so-called FD, a CD-ROM, a DVD disk, a magneto-optical disk, or an IC card, which is inserted in the computer 100. The computer 100 may then obtain the prediction control program 170a from the portable physical medium and execute the obtained prediction control program 170a. The prediction control program 170a may be stored beforehand in another computer or a server apparatus, for example, which is connected to the computer 100 via a public network, the Internet, a LAN, or a WAN. The prediction control program 170a thus stored may be executed by being downloaded onto the computer 100.

Diverse three-dimensional structures are able to be predicted.

## Claims

1. A prediction control program of a structure prediction model (11) that predicts a three-dimensional structure of an organic compound from sequence information on the organic compound, the prediction control program causing a computer (1) to execute a process comprising:

   changing an intermediate feature value of the structure prediction model so that a difference between first limiting information and second limiting information different from the first limiting information is lessened, the first limiting information corresponding to a predicted structure output as a prediction result from the structure prediction model.

2. The prediction control program according to claim 1, wherein the changing includes using a gradient obtained by backpropagation of the difference to change the intermediate feature value of the structure prediction model.

3. The prediction control program according to claim 1, wherein

   the changing includes converting the predicted structure to the first limiting information by using any one of an interpolation formula satisfying a density conservation law and an approximate equation according to a spherically symmetric Gaussian distribution, and
   the process further includes calculating the difference between the first limiting information and the second limiting information.

4. The prediction control program according to claim 3, wherein the calculating the difference includes calculating the difference by using any one of a cross-correlation, an L2 norm, or an L1 norm.

5. The prediction control program according to claim 4, wherein the calculating the difference further includes calculating an objective function having a second objective function added to a first objective function representing a constraint on the predicted structure using the difference, the second objective function indicating a constraint on the organic compound.

6. The prediction control program according to claim 5, wherein the calculating the difference includes using coarse and dense mixing for area segmentation, for the first objective function.

7. The prediction control program according to claim 1, wherein the process further includes:

   executing preprocessing of determining a rigid body transformation by which the predicted structure and the second limiting information are fitted to each other, and
   the changing includes applying the rigid body transformation to a predicted structure newly output as a prediction result from the structure prediction model.

8. The prediction control program according to claim 7, wherein

   the executing the preprocessing further includes finding, through point set registration, a rigid body transformation to a chain of the predicted structure in a case where the three-dimensional structure to be predicted is a multimer, and
   the changing includes applying the rigid body transformation to the predicted structure newly output as the prediction result from the structure prediction model.

9. The prediction control program according to claim 1, wherein the first limiting information and the second limiting information are any one of a three-dimensional density map derived from an electron microscope, a three-dimensional density map derived from X-ray analysis, or atomic coordinates of an all-atom structure model.

10. An information processing apparatus (1) that controls a structure prediction model (11) that predicts a three-dimensional structure of an organic compound from sequence information on the organic compound, the information processing apparatus comprising:
a control unit (10) configured to:
change an intermediate feature value of the structure prediction model so that a difference between first limiting information and second limiting information different from the first limiting information is lessened, the first limiting information corresponding to a predicted structure output as a prediction result from the structure prediction model.

11. A prediction control method that is a method of controlling a structure prediction model (11) that predicts a three-dimensional structure of an organic compound from sequence information on the organic compound, wherein a computer (1) executes a process comprising:
changing an intermediate feature value of the structure prediction model so that a difference between first limiting information and second limiting information different from the first limiting information is lessened, the first limiting information corresponding to a predicted structure output as a prediction result from the structure prediction model.

# FIG.1

CHANGE INTERMEDIATE FEATURE VALUES
*FREEZE TRAINING PARAMETERS

STRUCTURE PREDICTION MODEL — 11

INTERMEDIATE FEATURE VALUES

S6

BACKPROPAGA-TION

S5

AMINO ACID SEQUENCE

S1

Input sequence

Generic database search

Pairing

Structure database search

MSA

Templates

Single repr. (r, c)

Pair representation (r or r, c)

LAYER

High confidence

Low confidence

3D structure

ATOMIC STRUCTURE OF PROTEIN

S2, S3

m1

THREE-DIMEN-SIONAL DENSITY MAP

CONVERT IN DIFFERENTIABLE FORM

DIF-FER-ENCE

S4

m0

THREE-DIMEN-SIONAL DENSITY MAP ACTUALLY MEASURED

←Recycling (three times)

EP 4 607 518 A1

13

# FIG.2

INFORMATION PROCESSING APPARATUS — 1

CONTROL UNIT — 10

STORAGE UNIT — 20

AMINO ACID SEQUENCE

STRUCTURE PREDICTION MODEL — 11

UPDATE UNIT — 15

PRE-PROCESSING UNIT — 12

CONVERSION UNIT — 13

DIFFERENCE CALCULATION UNIT — 14

OUTPUT UNIT — 16

PDB FILE — 21

EMDB DATA — 22

EM DATA — 23

EP 4 607 518 A1

# FIG.3

# FIG.4

FITTING TO THREE-DIMENSIONAL DENSITY MAP

# FIG.5

# FIG.6

```
                        ┌─────────────────┐
                        │      START      │
                        └─────────────────┘
                                 │
                                 ▼                          S11
┌─────────────────────────────────────────────────────────────┐
│ PREDICT STRUCTURES OF MONOMERS FOR EACH CHAIN FROM AMINO ACID │
│                          SEQUENCE                             │
└─────────────────────────────────────────────────────────────┘
                                 │
                                 ▼                          S12
┌─────────────────────────────────────────────────────────────┐
│          PERFORM PREPROCESSING OF FINDING THREE RIGID BODY    │
│ TRANSFORMATIONS FITTING CHAINS OF STRUCTURES OF THREE MONOMERS│
│           (TRIMER) PREDICTED, BY USING PDB FILE               │
└─────────────────────────────────────────────────────────────┘
                                 │
                                 ▼                          S13
┌─────────────────────────────────────────────────────────────┐
│  USE RIGID BODY TRANSFORMATIONS FOUND FOR CHAINS AND PERFORM  │
│                 COMBINATION INTO ONE MULTIMER                 │
└─────────────────────────────────────────────────────────────┘
                                 │
                                 ▼                          S14
┌─────────────────────────────────────────────────────────────┐
│ PERFORM PREPROCESSING OF FINDING RIGID BODY TRANSFORMATIONS Rc│
│         AND tc FITTING LIMITING EM, FOR MULTIMER              │
└─────────────────────────────────────────────────────────────┘
                                 │
                                 ▼                          S15
┌─────────────────────────────────────────────────────────────┐
│ PERFORM FORWARD PROPAGATION THROUGH EACH LAYER FOR EACH CHAIN │
└─────────────────────────────────────────────────────────────┘
                                 │
                                 ▼                          S16
┌─────────────────────────────────────────────────────────────┐
│  APPLY RIGID BODY TRANSFORMATIONS FOUND BY PREPROCESSING AND  │
│          FIND ALL-ATOM STRUCTURE MODEL OF TRIMER             │
└─────────────────────────────────────────────────────────────┘
                                 │
                                 ▼                          S17
┌─────────────────────────────────────────────────────────────┐
│ CONVERT ATOMIC STRUCTURE OF TRIMER TO THREE-DIMENSIONAL DENSITY│
│                            MAP                               │
└─────────────────────────────────────────────────────────────┘
                                 │
                                 ▼                          S18
┌─────────────────────────────────────────────────────────────┐
│ FIND DIFFERENCE BETWEEN THREE-DIMENSIONAL DENSITY MAP AND EM  │
└─────────────────────────────────────────────────────────────┘
                                 │
                                 ▼                          S19
┌─────────────────────────────────────────────────────────────┐
│   CALCULATE OBJECTIVE FUNCTION BY USING DIFFERENCE FOUND     │
└─────────────────────────────────────────────────────────────┘
                                 │
                                 ▼                          S20
┌─────────────────────────────────────────────────────────────┐
│        PERFORM CALCULATION FOR BACKPROPAGATION              │
│ (S19→S18→S17→S16→S15) AND FIND INTERMEDIATE FEATURE VALUES   │
└─────────────────────────────────────────────────────────────┘
                                 │
                                 ▼                          S21
┌─────────────────────────────────────────────────────────────┐
│           UPDATE INTERMEDIATE FEATURE VALUES                 │
└─────────────────────────────────────────────────────────────┘
                                 │
                                 ▼                          S22
        NO          ◇ HAS CONVERGENCE BEEN REACHED? ◇
                                 │
                               YES
                                 ▼
                        ┌─────────────────┐
                        │       END       │
                        └─────────────────┘
```

- **S11**: PREDICT STRUCTURES OF MONOMERS FOR EACH CHAIN FROM AMINO ACID SEQUENCE
- **S12**: PERFORM PREPROCESSING OF FINDING THREE RIGID BODY TRANSFORMATIONS FITTING CHAINS OF STRUCTURES OF THREE MONOMERS (TRIMER) PREDICTED, BY USING PDB FILE
- **S13**: USE RIGID BODY TRANSFORMATIONS FOUND FOR CHAINS AND PERFORM COMBINATION INTO ONE MULTIMER
- **S14**: PERFORM PREPROCESSING OF FINDING RIGID BODY TRANSFORMATIONS $R_c$ AND $t_c$ FITTING LIMITING EM, FOR MULTIMER
- **S15**: PERFORM FORWARD PROPAGATION THROUGH EACH LAYER FOR EACH CHAIN
- **S16**: APPLY RIGID BODY TRANSFORMATIONS FOUND BY PREPROCESSING AND FIND ALL-ATOM STRUCTURE MODEL OF TRIMER
- **S17**: CONVERT ATOMIC STRUCTURE OF TRIMER TO THREE-DIMENSIONAL DENSITY MAP
- **S18**: FIND DIFFERENCE BETWEEN THREE-DIMENSIONAL DENSITY MAP AND EM
- **S19**: CALCULATE OBJECTIVE FUNCTION BY USING DIFFERENCE FOUND
- **S20**: PERFORM CALCULATION FOR BACKPROPAGATION (S19→S18→S17→S16→S15) AND FIND INTERMEDIATE FEATURE VALUES
- **S21**: UPDATE INTERMEDIATE FEATURE VALUES
- **S22**: HAS CONVERGENCE BEEN REACHED? (NO → returns to S15; YES → END)

# FIG.7

COMPUTER — 100

SPEAKER — 110b

OPERATION UNIT — 110a

CAMERA — 110c

CPU — 150

ROM — 160

COMMUNI-CATION UNIT — 130

BUS — 140

DISPLAY — 120

RAM — 180

PREDICTION CONTROL PROCESS — 180a

HDD — 170

PREDICTION CONTROL PROGRAM — 170a

EP 4 607 518 A1

FIG.8

Europäisches
Patentamt

European
Patent Office

Office européen
des brevets

# EUROPEAN SEARCH REPORT

**Application Number**

EP 25 15 7892

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JUMPER JOHN ET AL: "Highly accurate protein structure prediction with AlphaFold", NATURE,, vol. 596, no. 7873, 15 July 2021 (2021-07-15), pages 583-589, XP037548247, DOI: 10.1038/S41586-021-03819-2 [retrieved on 2021-07-15] | 1-3,5,6, 9-11 | INV. G16B15/20 G16B40/20 |
| Y | * Fig. 1e; page 585, col. 2; page 586, col. 2; page 587, col. 1; page 588, col. 2 * | 4,7,8 | |
| Y | CN 115 083 513 A (UNIV HUAZHONG SCIENCE TECH) 20 September 2022 (2022-09-20) * 3rd para. of Background section * | 4 | |
| Y | CHEN HUILING ET AL: "M-TASSER: An Algorithm for Protein Quaternary Structure Prediction", BIOPHYSICAL JOURNAL, vol. 94, no. 3, 1 February 2008 (2008-02-01), pages 918-928, XP029293715, ISSN: 0006-3495, DOI: 10.1529/BIOPHYSJ.107.114280 * page 920, col. 2 * | 7,8 | **TECHNICAL FIELDS SEARCHED (IPC)** G16B |

The present search report has been drawn up for all claims

| Place of search | Date of completion of the search | Examiner |
|---|---|---|
| Munich | 27 June 2025 | Schmidt, Karsten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 7892

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2025

| Patent document cited in search report | Publication date | Patent family member(s) | Publication date |
|---|---|---|---|
| CN 115083513 A | 20-09-2022 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000229994 A

**Non-patent literature cited in the description**

- **YOSUKE OYAMA** ; **AKIHIRO TABUCHI** ; **ATSUSHI TOKUHISA**. Accelerating AlphaFold2 Inference of Protein Three-Dimensional Structure on the Super-computer Fugaku. *FlexScience '23: Proceedings of the 13th Workshop on AI and Scientific Computing at Scale using Flexible Computing*, 11 August 2003, 1-9